# EUROPEAN PATENT APPLICATION

(11) **EP 2 617 358 A1**
(43) Date of publication of application: **24.07.2013**
(21) Application number: 10768986.1
(22) Date of filing: 16.09.2010
(51) Int. Cl.: A61B 6/00

(54) **X-RAY DEVICE STABILIZER**

(71) Applicant: Sociedad Española De Electromedicina Y Calidad, S. A., 28110 Algete (ES)
(72) Inventor: CAUSAPÉ RODRÍGUEZ, Andrés, E-28028 Madrid (ES); MORENO VALLEJO, Ildefonso, E-28521 Madrid (ES)
(74) Representative: Monzón de la Flor, Luis Miguel
(86) International application number: PCT/ES2010/070601
(87) International publication number: WO 2012/035176

(57) **Abstract**

The present invention is intended to a X-ray device stabilizer that allows X- ray images to be taken at positions far away from the car supporting such X-ray device, since it prevents the X-ray device from overturning when the X-ray tube is far away from the floor supporting points of the X-ray device.

## Description

### OBJECT OF THE INVENTION

The present invention is intended to a for X-ray device stabilizer that allows X-ray images to be taken at positions far away from the car supporting said X-ray device.

Due to its special configuration, the X-ray device stabilizer prevents the X-ray device from overturning when the X-ray tube is far away from the floor supporting points of the X-ray device.

### BACKGROUND OF THE INVENTION

In the state of the art are known X-ray devices having an X-ray tube for taking X-ray images to patients who are on a bed or horizontal surface.

Said X-ray devices are linked to a car with a pair of front wheels and a pair of rear wheels.

Among the previous devices is the one described in the patent with publication number S6851853, which also has a mast capable of rotating with respect to the car and a vertically movable support along said mast.

Fixed to the vertically movable support is a movable arm on which end opposite to that for fixing to the vertically movable support an X-ray tube and a collimator are arranged.

When a doctor examines a patient, the X-ray device is moved toward the bed wherein the patient is lying for sampling X-ray images.

In the previous device, the arm is telescopic in order to cover the distance between the car and the position in which the patient is on bed.

However, there is the drawback that when said distance between the car and the position in which the patient is on bed is very high, the bending moment due to the telescopic arm and X-ray tube and collimator weight is very high, which may result in the overturning of the X-ray device, with the consequent risk to both doctor and patient.

The present invention solves the previous drawbacks allowing X-ray images to be taken at positions far away from the car supporting the X-ray device, while the overturning of said X-ray device is prevented.

### DESCRIPTION OF THE INVENTION

The present invention relates to a X-ray device stabilizer that allows X-ray images to be taken at positions far away from the frame supporting said X-ray device without risk of overturning.

The X-ray device stabilizer is arranged over a frame positioned on the floor.

On the frame a mast capable of rotating with respect to the frame and a vertically movable support along said mast are arranged.

Fixed to the vertically movable support, and consequently to the mast is an arm on which end opposite to that for fixing to the vertically movable support an X-ray tube and a collimator for taking X-rays images to patients who are on a horizontal, vertical or inclined surface are arranged, because of the possibility of rotating the X-ray tube and collimator with respect to the arm.

The arm is telescopic, which allows covering the distance between the frame and the position wherein the patient is.

Connected to the mast is the stabilizer that comprises a cam for actuating at least one follower fixed to a movable base connected to the frame.

In this way the displacement of the movable base in the arm direction is carried out when the mast is rotated in order to position the X-ray tube and the collimator over the patient, consequently displacing the gravity center of the X-ray device toward the X-ray tube and thus preventing the frame from overturning.

When the mast is rotated in the longitudinal direction of the frame, forward or backward, the cam stops acting on the follower self-centered by recovery means disposed in said follower.

In summary, the invention relates to a X-ray device stabilizer comprising a frame on which a mast capable of rotating with respect to the frame is arranged, the mast having an arm on which end opposite to that for fixing to the mast an X-ray tube and a collimator for taking X-rays images to patients who are on a horizontal, vertical or inclined surface are arranged, characterized in that it comprises a cam connected to the mast which actuates a follower fixed to a movable base connected to the frame, so that the gravity center of the X-ray device is moved toward the X-ray tube.

### DESCRIPTION OF THE DRAWINGS

The present specification is complemented with a set of planes, for illustrating and not-limiting the preferred embodiment of the invention.
Figure 1 shows a plan view of the X-ray device stabilizer of the present invention when the detachable arm coincides with one of the main directions of the frame and contained in the projection thereof.
Figure 2 shows a perspective view of the X-ray device stabilizer shown in Figure 1

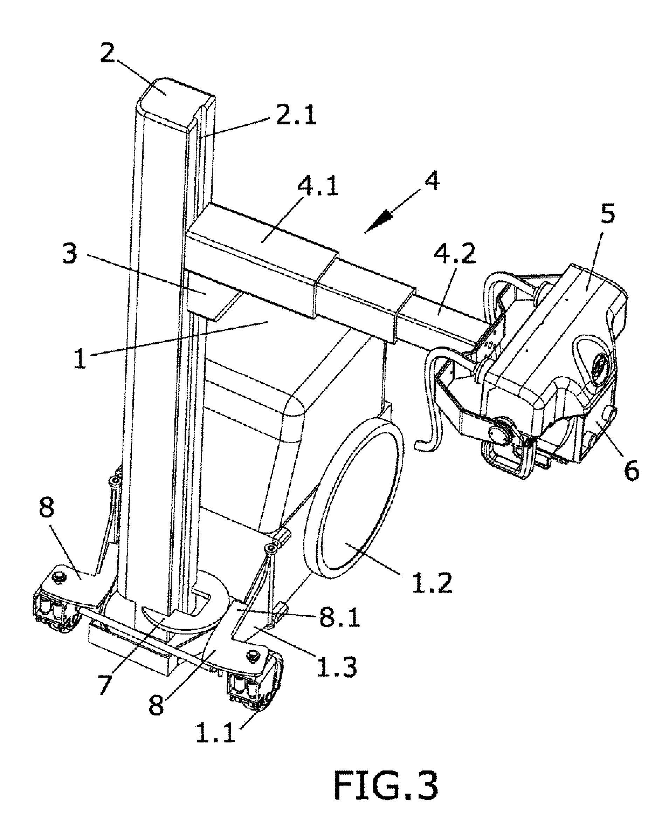

Figure 3 shows a perspective view wherein the detachable arm is rotated with respect to the longitudinal direction of the frame.
Figure 4 shows a perspective view of the X-ray device stabilizer of the present invention, wherein the detachable arm has been rotated with respect to the position occupied in Figure 2 and is projected with respect to the chassis.

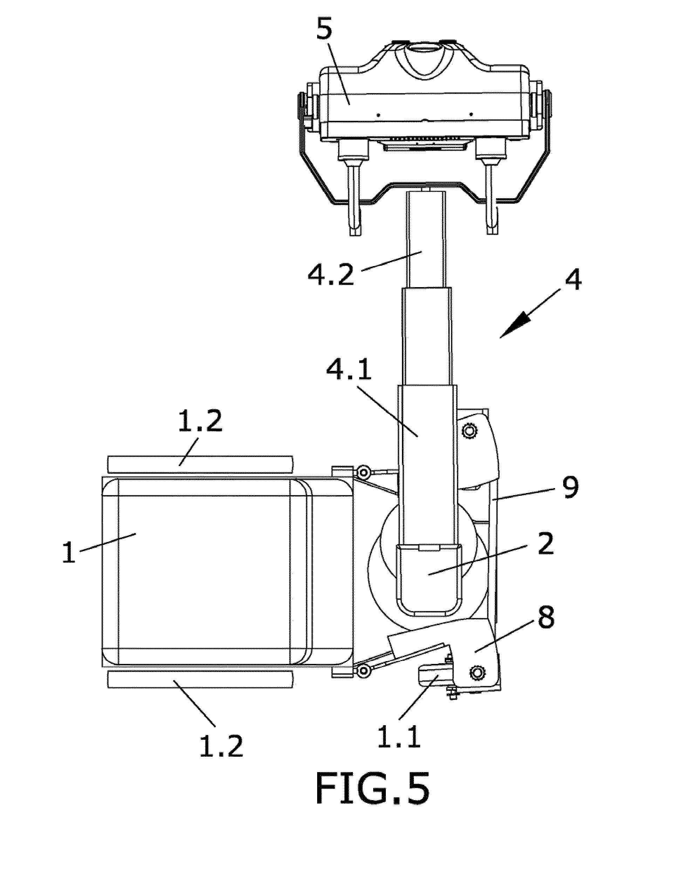

Figure 5 shows a plan view wherein the detachable arm is perpendicular to the longitudinal direction of the chassis.

### PREFERRED EMBODIMENT OF THE INVENTION

In view of the aforementioned, the present invention relates to a stabilizer for X-ray device which is arranged in a frame, which in this preferred embodiment example is a car (1) having four floor supporting points in the form of two front wheels (1.1) and two rear wheels (1.2).

On the car (1) a mast (2) capable of rotating with respect to the car (1) and a vertically moveable support (3) along a guide (2.1) in said mast (2) are arranged.

Fixed to the vertically movable support (3) is a telescopic arm (4) comprising three tubular sections, wherein the first tubular section (4.1) is fixed to the vertically movable support (3) and on the free end of the third tubular section (4.2) an X-ray tube (5) and a collimator (6) for taking X-ray images to patients who are on a bed (not shown) are arranged.

Connected to the mast (2) is the stabilizer comprising a circular cam (7) that actuates two followers (8) comprising elongated sections (8.1) between which the cam (7) for pushing one or another section (8.1) depending on the rotating direction established in the mast (1) is positioned.

These elongated sections (8.1) are fixed to flat bars (1.3) rotating with respect to a vertical axis that connects them to the car (1), while the followers (8) are hugely connected to the front wheels (1.1) of the car (1) constituting the movable base.

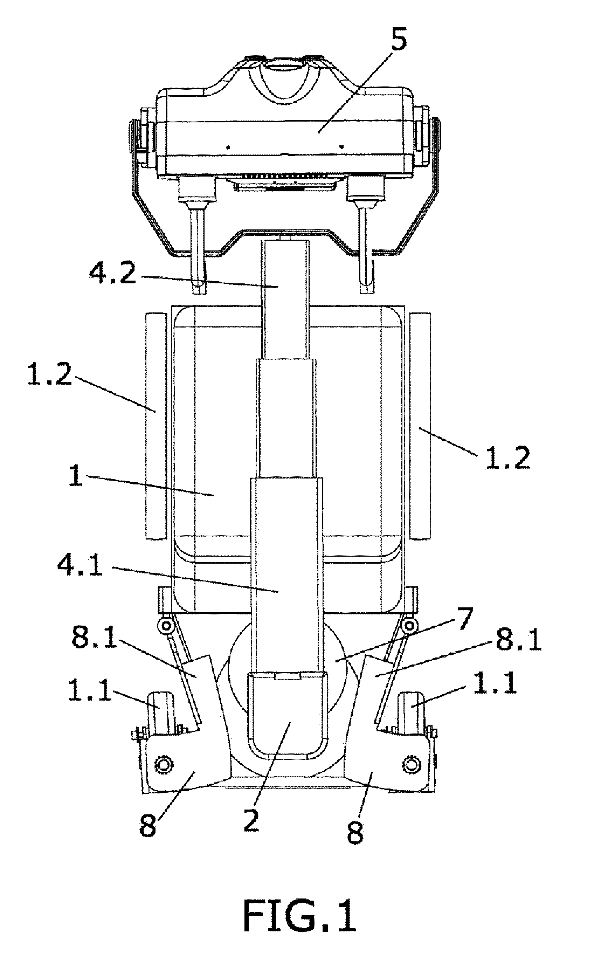

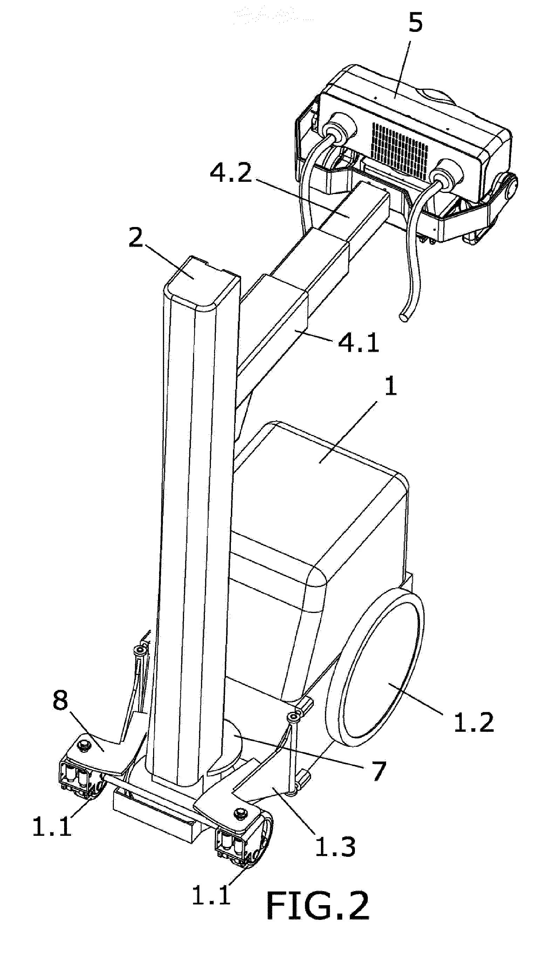

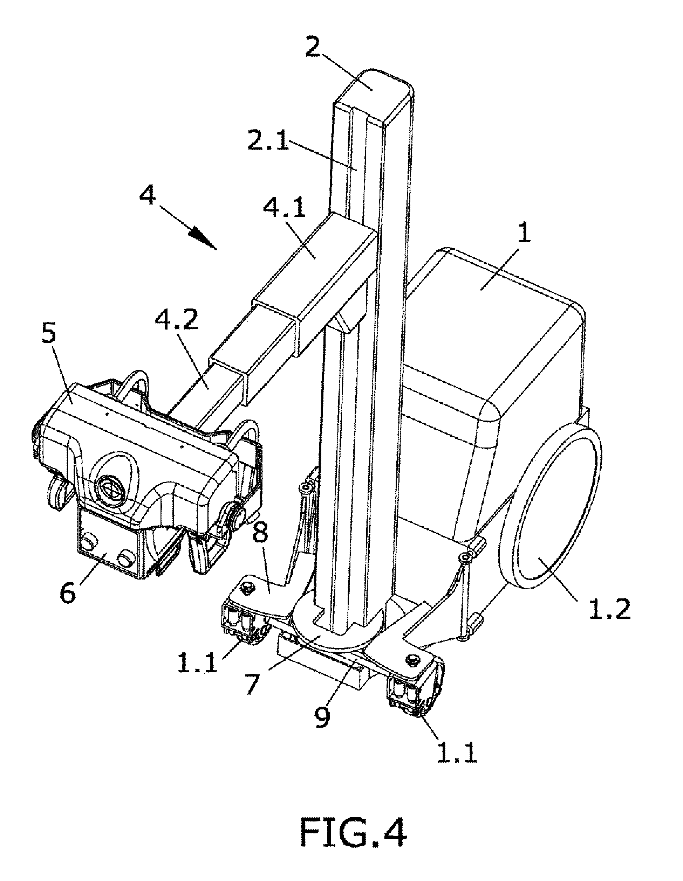

When the mast (2) is rotating in the longitudinal direction of the car (1), forward or backward, as shown in Figures 1, 2 and 4, the cam (7) stops acting on the followers (8) self- centered by recovery means (9) arranged between said followers, which in this example is a tension spring.

Variations on materials, shape, size and arrangement of the components described in a not limitative manner does not affect the essence of this invention, this being enough to proceed to its reproduction by an expert.

## Claims

1. X-ray device stabilizer comprising a frame on which a mast (2) capable of rotating with respect to the frame is arranged, the mast (2) having an arm (4) on which end opposite to that for fixing to the mast (2) an X-ray tube (5) and a collimator (6) for taking X-rays images to patients who are on a horizontal, vertical or inclined surface are arranged, **characterized in that** it comprises a cam (7) connected to the mast (2) which actuates at least one follower (8) fixed to a movable base connected to the frame, so that the gravity center of the X-ray device is moved toward the X-ray tube (5).

2. X-ray device stabilizer defined in claim 1 **characterized in that** it further comprises a vertically moveable support (3) along the mast (2).

3. X-ray device stabilizer defined in the preceding claims **characterized in that** the arm (4) is telescopic.

4. X-ray device stabilizer defined in any of the preceding claims **characterized in that** the frame is a car (1) having four floor supporting points in the form of two front wheels (1.1) acting as movable base and two rear wheels (1.2).

5. X-ray device stabilizer defined in claim 3 **characterized in that** the telescopic arm (4) comprises three tubular sections, wherein a first tubular section (4.1) is fixed to the vertically movable support (3) and on the free end of a third tubular section (4.2) an X-ray tube (5) and a collimator (6) are arranged.

6. X-ray device stabilizer defined in any of the preceding claims **characterized in that** the cam (7) is circular.

7. X-ray device stabilizer defined in any of the claim 4 **characterized in that** it has two followers (8) comprising elongated sections (8.1) between which the cam (7) for pushing one or another section (8.1) depending on the rotating direction established in the mast (1) is positioned.

8. X-ray device stabilizer defined in claim 7 **characterized in that** the elongated sections (8.1) are fixed to flat bars (1.3) rotating with respect to a vertical axis that connects them to the car (1), whereas the followers (8) are hingely connected to the front wheels (1.1) of the car.

9. X-ray device stabilizer defined in claim 7 **characterized in that** when the mast (2) is rotated in the longitudinal direction of the car (1), forward or backward, the cam (7) stops acting on the followers (8) self-centered by recovery means (9) arranged between said followers (8).
